# EUROPÄISCHE PATENTSCHRIFT

(11) **EP 2 408 738 B1**
(45) Veröffentlichungstag und Bekanntmachung des Hinweises auf die Patenterteilung: **26.07.2017**
(21) Anmeldenummer: 10709217.3
(22) Anmeldetag: 18.03.2010
(51) Int. Cl.: C07C 263/10, C07C 265/00, C07B 43/10, F28D 7/00

(54) **VERFAHREN ZUR HERSTELLUNG VON ISOCYANATEN**
METHOD FOR PRODUCING ISOCYANATES
PROCÉDÉ DE PRÉPARATION D'ISOCYANATES

(30) Priorität: 20.03.2009 EP 09155751
(43) Veröffentlichungstag der Anmeldung: 25.01.2012
(73) Patentinhaber: BASF SE, 67056 Ludwigshafen am Rhein (DE)
(72) Erfinder: MATTKE, Torsten, 67251 Freinsheim (DE); KNÖSCHE, Carsten, 67150 Niederkirchen (DE); RUMPF, Bernd, 68766 Hockenheim (DE); STROEFER, Eckhard, 68163 Mannheim (DE)
(86) Internationale Anmeldenummer: PCT/EP2010/053527
(87) Internationale Veröffentlichungsnummer: WO 2010/106131

(56) Entgegenhaltungen:
- EP-A1- 1 275 639
- EP-A1- 1 616 857
- EP-A1- 2 062 876
- EP-A2- 1 319 655
- EP-A2- 1 754 698
- EP-B1- 1 449 826
- WO-A1-2010/100221
- WO-A2-2010/052230

## Beschreibung

Die Erfindung betrifft ein Verfahren zur Herstellung von Isocyanaten durch Umsetzung der korrespondierenden Amine mit Phosgen in der Gasphase, gegebenenfalls in Gegenwart eines Inertmediums, bei dem Phosgen und Amin zunächst verdampft, dann weiter auf Reaktionstemperatur überhitzt werden und das überhitzte Phosgen und Amin gemischt und einem Reaktor zugeführt werden, in dem das Phosgen und das Amin zum Isocyanat umgesetzt werden. Die Herstellung von Isocyanaten durch Phosgenierung der korrespondierenden Amine kann prinzipiell durch eine Flüssigphasen- oder eine Gasphasenphosgenierung erfolgen. Die Gasphasenphosgenierung zeichnet sich dadurch aus, dass eine höhere Selektivität, ein geringerer Hold-up an toxischem Phosgen sowie ein verminderter Energiebedarf erforderlich sind.

Bei der Gasphasenphosgenierung werden ein aminhaltiger Eduktstrom und ein phosgenhaltiger Eduktstrom jeweils im gasförmigen Zustand vermischt. Das Amin und das Phosgen setzen sich unter Freisetzung von Chlorwasserstoff (HCl) zu den entsprechenden Isocyanaten um. Der aminhaltige Eduktstrom liegt im Allgemeinen in flüssiger Phase vor und muss vor der Vermischung mit dem phosgenhaltigen Strom verdampft und gegebenenfalls überhitzt werden.

Entsprechende Verfahren zur Herstellung von Isocyanaten in der Gasphase sind zum Beispiel in EP-A 1 319 655 oder EP-A 1 555 258 oder EP 1 754 698 oder EP 1 449 826 beschrieben. Die Verdampfung und Überhitzung von Amin und Phosgen auf Reaktionstemperatur kann entweder durch Totalverdampfung des jeweiligen benötigten Eduktstroms oder aber durch Teilverdampfung eines Flüssigstroms mit Rückführung des verbliebenen kondensierten Anteils auf dem Verdampfereingang erfolgen.

Insbesondere bei der Überhitzung des Phosgens kann es jedoch gemäß des entsprechenden chemischen Gleichgewichts zur Rückspaltung des Carbaminsäurechlorids in Chlor und Kohlenmonoxid kommen. So liegen zum Beispiel nach Atkinson et al., J. Chem. Soc. Trans. 117, Vol. II, 1920, Seite 1410, bei 210°C und Normaldruck rund 0,2% des Phosgens dissoziiert vor. Bei 355°C liegen jedoch schon etwa 10% dissoziiert vor. Das im Phosgenstrom enthaltene Chlor hat jedoch den Nachteil, dass im Reaktor bei einer Reaktionstemperatur im Bereich von 360 bis 450°C das Isocyanat chloriert wird. Dies führt jedoch zu Qualitätsproblemen. So werden z.B. bei der Herstellung von Hexamethylendiisocyanat chlorierte Komponenten für Farbprobleme des Produkts verantwortlich gemacht.

Aufgabe der vorliegenden Erfindung ist es daher, ein Verfahren zur Herstellung von Isocyanaten durch Umsetzung der korrespondierenden Amine mit Phosgen bereitzustellen, bei dem eine Rückspaltung des Carbaminsäurechlorids in Chlor und Kohlenmonoxid reduziert oder sogar verhindert wird.

Gelöst wird die Aufgabe durch ein Verfahren zur Herstellung von Isocyanaten durch Umsetzung der korrespondierenden Amine mit Phosgen in der Gasphase, gegebenenfalls in Gegenwart eines Inertmediums, bei dem Phosgen und Amin zunächst verdampft und dann weiter auf Reaktionstemperatur erhitzt werden, das überhitzte Phosgen und Amin gemischt und einem Reaktor zugeführt werden, in dem das Phosgen und das Amin zum Isocyanat umgesetzt wird, wobei die Verweilzeit des Phosgens bei Temperaturen größer als 300°C maximal 5 s beträgt. Die Verweilzeit des Phosgens in Anlagenteilen mit hoher Temperatur, das heißt einer Temperatur von mehr als 300°C von weniger als 5 s, bevorzugt weniger als 2,5 s, hat den Vorteil, dass aufgrund der dadurch realisierten geringen Temperaturbelastung die Dissoziation des Phosgens in Chlor und Kohlenmonoxid reduziert werden kann.

Eine Verweilzeit des Phosgens von weniger als 5 s, bevorzugt von weniger als 2,5 s bei Temperaturen von mehr als 300°C wird zum Beispiel durch eine schnellere Überhitzung des Phosgens erzielt. Eine schnellere Überhitzung wird beispielsweise erzielt, wenn die Verdampfung und Überhitzung des Phosgens in einem gemeinsamen Wärmeübertrager mit einem Verhältnis von Verdampferoberfläche zu Volumen von mehr als 750 1/m durchgeführt werden. Durch das Verhältnis von Verdampferoberfläche zu Volumen von mehr als 750 1/m wird ein schneller Wärmeübergang und damit eine schnelle Erwärmung des Phosgens erreicht. Hierdurch kann ebenfalls die Verweilzeit reduziert werden und die Dissoziation von Phosgen in Kohlenmonoxid und Chlor reduziert werden, um so den Chlorgehalt im Eduktstrom zu minimieren.

Da die Dissoziation des Phosgens in Chlor und Kohlenmonoxid mit steigender Temperatur zunimmt, ist es weiterhin bevorzugt, das Phosgen auf eine Temperatur von weniger als 500 °C, bevorzugt weniger als 450 °C und insbesondere weniger als 400 °C zu überhitzen. Durch diese Maßnahme lässt sich ebenfalls die Dissoziation des Phosgens in Chlor und Kohlenmonoxid reduzieren.

Die für die Reaktion von Phosgen und Amin zum Isocyanat notwendige Temperatur liegt im Allgemeinen im Bereich von 250 bis 550°C, insbesondere im Bereich von 300 bis 500°C. Der Druck, bei dem die Reaktion durchgeführt wird, liegt vorzugsweise im Bereich zwischen 0,3 bis 3 bar absolut. Besonders bevorzugt im Bereich von 0,8 bis 3,0 bar absolut.

Die Erwärmung auf die erforderliche Reaktionstemperatur erfolgt vorzugsweise vor der Vermischung von Phosgen und Amin, da zur Vermeidung von Reaktionsnebenprodukten eine kurze Verweilzeit des Reaktionsgemisches bei Reaktionstemperatur gewünscht ist, um eine Zersetzung oder weitere Reaktion des Isocyanats zu vermeiden.

Die Erwärmung des Phosgens und des Amins vor deren Vermischung kann z.B. durch eine elektrische Beheizung oder eine direkte oder indirekte Aufheizung durch Verbrennung eines Brennstoffs oder auch durch Wärmetausch mit einem Heizmedium erfolgen. Wenn die Aufheizung durch Verbrennung eines Brennstoffs erfolgt, so werden üblicherweise Brenngase, beispielsweise Erdgas, eingesetzt. Für die Erwärmung mit einem Heizmedium eignen sich z.B. Wärmeträgeröle oder auch Heizdampf. Bei der Verwendung von Heizdampf erfolgt üblicherweise eine mehrstufige Erwärmung, wobei Heizdampf mit unterschiedlichem Druck und unterschiedlichen Temperaturen eingesetzt werden kann. Die Überhitzung des Phosgens und des Amins erfolgt dann jedoch üblicherweise mit einem Wärmeträger Öl oder beispielsweise durch elektrische Beheizung oder direkte oder indirekte Aufheizung durch Verbrennung eines Brennstoff. Wenn zur Verdampfung des Phosgens und Erwärmung des Amins Wasserdampf eingesetzt wird, so liegt der Dampfdruck des Wasserdampfs z.B. im Bereich von 40 bis 100 bar. Daraus ergibt sich eine Temperatur des Wasserdampfs im Bereich von 250 bis 311°C, sofern kein überhitzter Wasserdampf eingesetzt wird.

Bei der Verdampfung und Überhitzung des Phosgens und des Amins sind Hotspots zu vermeiden. Besonders bevorzugt ist daher die Verdampfung und Überhitzung durch Verwendung eines Heizmediums.

Der Reaktor, der zur Phosgenierung des Amins zur Herstellung von Isocyanaten eingesetzt wird, ist dem Fachmann bekannt. Im Allgemeinen werden als Reaktoren Rohrreaktoren eingesetzt. Im Reaktor wird das Amin mit dem Phosgen zum korrespondierenden Isocyanat und Chlorwasserstoff umgesetzt. Üblicherweise wird das Phosgen im Überschuss zugegeben, so dass das im Reaktor entstehende Reaktionsgas neben dem gebildeten Isocyanat und dem Chlorwasserstoff auch Phosgen enthält.

Amine, die zur Herstellung von Isocyanaten eingesetzt werden können, sind Monoamine, Diamine, Triamine oder höherwertige Amine. Bevorzugt werden Monoamine oder Diamine eingesetzt. Entsprechend des eingesetzten Amins ergeben sich die korrespondierenden Monoisocyanate, Diisocyanate, Triisocyanate oder höherwertigen Isocyanate. Bevorzugt werden Monoisocyanate oder Diisocyanate mit dem erfindungsgemäßen Verfahren hergestellt.

Diamine und Diisocyanate können aliphatisch, cycloaliphatisch oder aromatisch sein.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

Im Folgenden wird die Bezeichnung (cyclo)aliphatische Isocyanate für cycloaliphatische und/oder aliphatische Isocyanate verwendet.

Beispiele für aromatische Mono- und Diisocyanate sind bevorzugt solche mit 6 bis 20 C-Atomen, beispielsweise Phenylisocyanat, monomeres 2,4'- und/oder 4,4'-Methylen-di(phenylisocyanat) (MDI), 2,4- und/oder 2,6-Toluylendiisocyanat (TDI) und 1,5- oder 1,8-Naphthyldiisocyanat (NDI).

Beispiele für (cyclo)aliphatische Diisocyanate sind aliphatische Diisocyanate wie 1,4-Tetramethylendiisocyanat, 1,6-Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, 1,5-Diisocyanatopentan, Neopentandiisocyanat, Derivate des Lysindiisocyanats, Tetramethylxylylendiisocyanat (TMXDI), Trimethylhexandiisocyanat oder Tetramethylhexandiisocyanat, sowie 3(beziehungsweise 4)-8(beziehungsweise 9)-Bis(isocyanatomethyl)-tricyclo-[5.2.1.0^{2.6}]-decan-Isomerengemische, sowie cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5-tri-methyl-5-(isocyanatomethyl)cyclohexan (Isoforondiisocyanat), 1,3- oder 1,4-Bis(isocyanatomethyl)cyclohexan, 2,4- oder 2,6-Diisocyanato-1-methylcyclohexan.

Bevorzugte (cyclo)aliphatische Diisocyanate sind 1,6-Diisocyanatohexan, 1-Isocyanato-3,3,5-trimethyl-5-(isocyanatomethyl)cyclohexan und 4,4'-Di(isocyanato-cyclohexyl)methan. Besonders bevorzugt sind 1,6-Diisocyanatohexan, 1-Isocyanato-3,3,5-trimethyl-5-(isocyanatomethyl)cyclohexan, 1,5-Diisocyanatopentan und 4,4'-Di(isocyanatocyclohexyl)methan.

Beispiele für aromatische Diisocyanate sind 2,4-, 2,6-Toluylendiisocyanat, Methylendiphenylisocyanat oder Isomerengemische davon.

Amine, die bei dem erfindungsgemäßen Verfahren zur Reaktion zu den korrespondierenden Isocyanaten eingesetzt werden, sind solche, bei denen das Amin, die korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 Mol-%, besonders bevorzugt zu höchstens 1 Mol-% und ganz besonders bevorzugt zu höchstens 0,5 Mol-% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1,5-Diaminopentan, 1,3-Bis(aminomethyl)cyclohexan, 1-Amino-3,3,5-trimethyl-5-amino-methylcyclohexan (IPDA) und 4,4-Diaminodicyclohexylmethan. Bevorzugt verwendet werden 1,6-Diaminohexan (HDA) und 1,5-Diaminopentan.

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (Mol/Mol)-Gemisch, Diaminobenzol, 2,6-Xylidin, Naphthyldiamin (NDA) und 2,4'- oder 4,4'-Methylen(diphenyldiamin) (MDA) oder Isomerengemische davon. Bevorzugt sind unter diesen die Diamine, besonders bevorzugt sind 2,4- und/oder 2,6-TDA oder 2,4'- und/oder 4,4'-MDA.

Zur Herstellung von Monoisocyanaten können ebenfalls aliphatische, cycloaliphatische oder aromatische Amine, üblicherweise Monoamine, eingesetzt werden. Als aromatisches Monoamin ist insbesondere Anilin bevorzugt.

Bei der Gasphasenphosgenierung ist es anzustreben, dass die im Reaktionsverlauf auftretenden Verbindungen, also Edukte (Amin und Phosgen), Zwischenprodukte (insbesondere die intermediär entstehenden Mono- und Dicarbamoylchloride), Endprodukte (Isocyanat), sowie gegebenenfalls zudosierte inerte Verbindungen, unter den Reaktionsbedingungen in der Gasphase verbleiben. Sollten diese oder andere Komponenten sich aus der Gasphase zum Beispiel an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für Auftreten der Aminhydrochloride, die sich aus freien Aminogruppen und Chlorwasserstoff bilden, da die resultierenden Aminhydrochloride leicht ausfallen und nur schwer wieder verdampfbar sind.

Neben dem Einsatz eines Rohrreaktors ist es auch möglich, im Wesentlichen quaderförmige Reaktionsräume, beispielsweise Plattenreaktoren zu verwenden. Auch jeder beliebige andere Querschnitt des Reaktors ist möglich.

Um die Bildung von Nebenprodukten zu vermeiden, ist es bevorzugt, Phosgen im Überschuss zuzuführen. Um nur den für die Reaktion notwendigen Anteil an Aminen zuzuführen, ist es möglich, das Amin mit einem Inertgas zu mischen. Durch den Anteil an Inertgas im Amin lässt sich die Menge des zugeführten Amins bei vorgegebener Geometrie der Zufuhröffnungen für das Amin und das Phosgen einstellen. Inertmedien, die zugegeben werden können, sind solche, die im Reaktionsraum gasförmig vorliegen und nicht mit den im Reaktionsverlauf auftretenden Verbindungen reagieren. Als Inertmedium können zum Beispiel Stickstoff, Edelgase wie Helium oder Argon, Aromaten wie Chlorbenzol, o-Dichlorbenzol, Trichlorbenzol, Toluol, Xylol, Chlornaphthalin, Decahydronaphthalin, Kohlenstoffdioxid oder Kohlenstoffmonoxid eingesetzt werden. Bevorzugt werden jedoch Stickstoff und/oder Chlorbenzol als Inertmedium verwendet.

Alternativ ist es jedoch auch möglich, zum Beispiel um einen zu großen Überschuss an Phosgen zu vermeiden, das Inertmedium dem Phosgen zuzumischen.

Im Allgemeinen wird das Inertmedium in einer Menge zugesetzt, dass das Verhältnis der Gasvolumina von Inertmedium zu Amin beziehungsweise zu Phosgen weniger als 0,0001 bis 30, bevorzugt weniger als 0,01 bis 15 und besonders bevorzugt weniger als 0,1 bis 5 beträgt.

Um die Bildung unerwünschter Nebenprodukte zu reduzieren beziehungsweise zu vermeiden und weiterhin auch eine Zersetzung des gebildeten Isocyanats zu unterbinden, wird das Reaktionsgas unmittelbar nach der Reaktion in einem Quench abgekühlt. Hierzu wird ein vorzugsweise flüssiges Quenchmedium zugegeben. Durch Erwärmung oder Verdampfung des Quenchmediums nimmt dieses Wärme auf und führt zu einer schnellen Abkühlung des Reaktionsgases.

Die Mischung von Phosgen und Amin erfolgt z.B. in einer Mischdüse, mit der Amin und Phosgen dem Reaktor zugeführt werden. Alternativ ist es auch möglich, Amin und Phosgen über geeignete Düsen einer Mischkammer zuzuführen, in der diese gemischt werden und dann in den Reaktor weiter strömen. Bevorzugt ist jedoch die Verwendung einer Mischdüse.

Um die gewünschte geringe Verweilzeit des Phosgens bei hohen Temperaturen, das heißt bei Temperaturen von mehr als 300°C von weniger als 5 Sekunden zu erzielen, können verschiedene Wärmeübertragertypen, beispielsweise Mikro- oder Milliwärme-übertrager, Rohrbündelwärmeübertrager, Wirbelschichtwärmeübertrager, Mikrowellenüberhitzer oder Wärmestrahler eingesetzt werden. Insbesondere sind Wärmeübertrager mit einer volumenspezifischen Verdampferoberfläche von mehr als 750 1/m bevorzugt, da die Verweilzeit des Phosgens bei hohen Temperaturen in derartigen Wärmeübertragern gering gehalten werden kann. Des Weiteren kann die Temperaturdifferenz zwischen Übertrageroberfläche und dem Phosgenstrom minimiert werden. Besonders vorteilhaft ist der Einsatz von Mikrowärmeübertragern, da Phosgen kaum Ablagerungen bildet, die zu Verstopfungen des Mikrowärmeübertragers führen können.

Bevorzugt zur Verdampfung und Überhitzung des Phosgens wird jedoch mindestens ein Rohrbündelwärmeübertrager eingesetzt. Der Rohrbündelwärmeübertrager kann mit oder ohne turbulenzerzeugende Ein- oder Anbauten ausgerüstet sein. Wenn das Phosgen in den Rohren des Rohrbündelwärmeübertragers geführt wird, eignen sich als Einbauten z.B. Drallgänge, erhöhte Wandrauhigkeiten, hiTRAN-Elemente, Drahtnetzwerke, Twisted Tapes oder andere, dem Fachmann bekannte Turbulenzerzeuger. Sollte das Phosgen die Rohre umströmen, können z.B. Fins oder Rippen eingesetzt werden. Durch die Verwendung turbulenzerhöhender Einbauten werden die notwendige Wandüberhitzung und die erforderliche Verweilzeit zur Überhitzung des Phosgens herabgesetzt. Auf diese Weise sinkt die Temperaturbelastung des Phosgenstroms.

In einer Ausführungsform wird ein quer angeströmtes Heizregister zur Überhitzung des Phosgens eingesetzt. Die Rohre des quer angeströmten Heizregisters können berippt oder unberippt ausgeführt werden. Auch ist es denkbar, die Überhitzung in mehreren Heizregistern in Stufen durchzuführen. Zur Strömungsvergleichmäßigung können zwischen den einzelnen Heizregistern Packungen installiert werden.

Ebenfalls zur Verdampfung und Überhitzung des Phosgens eignen sich Wendelrohre. Die in Wendelrohren erzeugten Sekundärströmungen bewirken einen hohen Wärmeübergangskoeffizienten und damit geringe Wandtemperaturen und Verweilzeiten. Auch können in einem Wendelrohr zusätzliche Einbauten zur Verbesserung des Wärmeübergangs, beispielsweise Turbulenzerzeuger, eingesetzt werden.

Neben Rohrbündelwärmeübertragern lassen sich auch Plattenwärmeübertrager einsetzen. In diesem Fall werden insbesondere Thermoplattenwärmeübertrager verwendet. Auch bei Einsatz von Thermoplattenwärmeübertragern können zur Intensivierung des Wärmeübergangs und damit zur Reduzierung der Wandtemperaturen und der Verweilzeiten Einbauten verwendet werden.

Weitere geeignete Wärmeübertrager sind z.B. Wirbelschichtwärmeübertrager, Mikrowellenüberhitzer und Wärmestrahler. Auch diese Bauformen erlauben jeweils geringe Wandüberhitzungen und kurze Verweilzeiten des Phosgens und ermöglichen so eine Reduzierung der Dissoziation des Phosgens in Chlor und Kohlenmonoxid.

Eine zur Herstellung von Isocyanaten durch Umsetzung der korrespondierenden Amine mit Phosgen in der Gasphase, ggf. in Gegenwart eines Inertmediums, geeignete Vorrichtung, umfasst einen Reaktor, eine Vorrichtung zur Mischung von Amin und Phosgen, die in den Reaktor mündet, sowie Vorrichtungen zur Verdampfung und zur Überhitzung von Phosgen und Amin. Die Vorrichtung zur Verdampfung und/oder Überhitzung des Phosgens ist dabei ein Wärmeübertrager mit einem Verhältnis von Verdampferoberfläche zum Volumen von mehr als 750 1/m.

Wie zuvor bereits beschrieben, kann durch einen Wärmeübertrager mit einem Verhältnis von Verdampferoberfläche zu Volumen von mehr als 750 1/m eine Verweilzeit des Phosgens von weniger als 5 Sekunden bei Temperaturen von mehr als 300°C erzielt werden. Hierdurch lässt sich die Dissoziation des Phosgens in Kohlenmonoxid und Chlor reduzieren und die Produktqualität des herzustellenden Isocyanats verbessern, da die Bildung von Nebenprodukten durch Reaktion mit Chlor reduziert wird.

Geeignete Wärmeübertrager, die ein Verhältnis von Verdampferoberfläche zu Volumen von mehr als 750 1/m aufweisen, sind z.B. Milli- oder Mikrowärmeübertrager.

Bevorzugt wird zur Verdampfung und/oder Überhitzung des Phosgens jedoch ein Rohrbündelwärmeübertrager eingesetzt, der ein Verhältnis von Verdampferoberfläche zu Volumen von mehr als 750 1/m aufweist. Das entsprechende Verhältnis kann z.B. bei Durchströmung der Rohre des Rohrbündelwärmeübertragers mit dem zu erhitzenden Phosgen durch den Rohrdurchmesser und gegebenenfalls Einbauten in den Rohren erzielt werden. Zur Verbesserung des Wärmeübergangs ist es, wie vorstehend bereits beschrieben, bevorzugt, wenn in den Rohren Turbulenzerzeuger angeordnet sind.

Um die Verweilzeit des Phosgens bei hohen Temperaturen zu reduzieren, ist es bevorzugt, wenn die Verdampfung und Überhitzung des Phosgens im gleichen Wärmeübertrager durchgeführt wird. Hierdurch können Rohrleitungen zwischen einzelnen Verdampfern eingespart werden und die Wege verkürzt werden. Dies führt gleichzeitig zu einer Verringerung der Verweilzeit. Zur schonenden Verdampfung und Überhitzung des Phosgens ist es bevorzugt, wenn der Wärmeübertrager mehrere Heizregister aufweist, in denen das Phosgen stufenweise verdampft und überhitzt wird. Die Heizregister können mit unterschiedlichen Heizmedien betrieben werden. So ist es z.B. zur Verdampfung des Phosgens möglich, ein Heizregister einzusetzen, das von Wasserdampf mit einem Druck von ca. 4 bar als Heizmedium durchströmt wird. An dieses kann sich ein weiteres Heizregister anschließen, das z.B. von Wasserdampf mit einem Druck im Bereich von 16 bis 40 bar durchströmt wird. Der Wasserdampf mit einem Druck im Bereich von 16 bis 40 bar, weist üblicherweise eine höhere Temperatur auf als der Wasserdampf von 4 bar, so dass hierdurch eine weitere Erwärmung erreicht wird. Daran kann sich ein weiteres Heizregister anschließen, das z.B. von einem Wärmeträgeröl durchströmt wird. Der Einsatz von Wärmeträgerölen erlaubt üblicherweise höhere Temperaturen als der Einsatz von Dampf. Auch ist es möglich, z.B. eine ionische Flüssigkeit oder eine Salzschmelze als Heizmedium zu verwenden. Vorteil des Einsatzes eines Heizmediums ist insbesondere, dass Hotspots und damit lokale Überhitzungen, die zu einer schnellen Spaltung des Phosgens in Chlor und Kohlenmonoxid führen können, vermieden werden.

Eine weitere Reduktion der Verweilzeit wird auch dadurch erzielt, wenn sich die Vorrichtung zur Mischung von Phosgen und Amin direkt an die Vorrichtung zur Überhitzung des Phosgens anschließt. Auf diese Weise können ebenfalls durchströmte Rohrleitungen eingespart werden und damit die Strömungszeit und die Verweilzeit des Phosgens bei Reaktionstemperatur verringert werden.

## Patentansprüche

1. Verfahren zur Herstellung von Isocyanaten durch Umsetzung der korrespondierenden Amine mit Phosgen in der Gasphase, gegebenenfalls in Gegenwart eines Inertmediums, bei dem Phosgen und Amin zunächst verdampft und dann weiter auf Reaktionstemperatur überhitzt werden, das überhitzte Phosgen und Amin gemischt und einem Reaktor zugeführt werden, in dem das Phosgen und das Amin zum Isocyanat umgesetzt werden, **dadurch gekennzeichnet, dass** die Verweilzeit des Phosgens bei Temperaturen größer als 300°C maximal 5 s beträgt.

2. Verfahren gemäß Anspruch 1, **dadurch gekennzeichnet, dass** die Verdampfung und/oder die Überhitzung des Phosgens in einem Mikro- oder Milliwärmeübertrager, einem Rohrbündelwärmeübertrager, einem Wirbelschichtwärmeübertrager, einem Mikrowellenüberhitzer oder einem Wärmestrahler erfolgen.

3. Verfahren gemäß Anspruch 1 oder 2, **dadurch gekennzeichnet, dass** die Verdampfung und/oder die Überhitzung des Phosgens in dem gleichen Apparat durchgeführt werden.

4. Verfahren gemäß Anspruch 1 oder 2, **dadurch gekennzeichnet, dass** die Verdampfung und die Überhitzung des Phosgens in mehreren Stufen erfolgen.

5. Verfahren gemäß einem der Ansprüche 1 bis 4, **dadurch gekennzeichnet, dass** das Phosgen dem Reaktor im Überschuss zugegeben wird.

6. Verfahren gemäß einem der Ansprüche 1 bis 5, **dadurch gekennzeichnet, dass** das dem Reaktor zugegebene Amin mit einem Inertgas gemischt ist.

## Claims

1. A process for preparing isocyanates by reacting the corresponding amines with phosgene in the gas phase, optionally in the presence of an inert medium, in which phosgene and amine are first evaporated and then superheated further to reaction temperature, and the superheated phosgene and amine are mixed and fed to a reactor in which the phosgene and the amine are converted to the isocyanate, wherein the residence time of the phosgene at temperatures greater than 300°C is not more than 5 s.

2. The process according to claim 1, wherein the phosgene is evaporated and/or superheated in a micro or milli heat transferer, a tube bundle heat transferer, a fluidized bed heat transferer, a microwave superheater or a heat radiator.

3. The process according to claim 1 or 2, wherein the evaporation and/or the superheating of the phosgene is/are performed in the same apparatus.

4. The process according to claim 1 or 2, wherein the evaporation and the superheating of the phosgene are effected in a plurality of stages.

5. The process according to any one of claims 1 to 4, wherein the phosgene is added to the reactor in excess.

6. The process according to any one of claims 1 to 5, wherein the amine added to the reactor is mixed with an inert gas.

## Revendications

1. Procédé de fabrication d'isocyanates par mise en réaction des amines correspondantes avec du phosgène dans la phase gazeuse, éventuellement en présence d'un milieu inerte, selon lequel le phosgène et l'amine sont tout d'abord évaporés, puis surchauffés à la température de réaction, le phosgène et l'amine surchauffés sont mélangés et introduits dans un réacteur, dans lequel le phosgène et l'amine sont transformés en l'isocyanate, **caractérisé en ce que** le temps de séjour du phosgène à des températures supérieures à 300 °C est d'au plus 5 s.

2. Procédé selon la revendication 1, **caractérisé en ce que** l'évaporation et/ou le surchauffage du phosgène ont lieu dans un échangeur de chaleur micro- ou millimétrique, un échangeur de chaleur à faisceau de tubes, un échangeur de chaleur à lit fluidisé, un surchauffeur à micro-ondes ou un émetteur de chaleur.

3. Procédé selon la revendication 1 ou 2, **caractérisé en ce que** l'évaporation et/ou le surchauffage du phosgène sont réalisés dans le même appareil.

4. Procédé selon la revendication 1 ou 2, **caractérisé en ce que** l'évaporation et le surchauffage du phosgène ont lieu en plusieurs étapes.

5. Procédé selon l'une quelconque des revendications 1 à 4, **caractérisé en ce que** le phosgène est ajouté en excès dans le réacteur.

6. Procédé selon l'une quelconque des revendications 1 à 5, **caractérisé en ce que** l'amine ajoutée au réacteur est mélangée avec un gaz inerte.
